**Europäisches Patentamt**

**European Patent Office**

**Office européen des brevets**

(19)

(11) Veröffentlichungsnummer : **0 117 977**
**B1**

(12) # EUROPÄISCHE PATENTSCHRIFT

(45) Veröffentlichungstag der Patentschrift :
12.11.86

(51) Int. Cl.⁴ : **A 01 J 7/00, G 01 N 1/10**

(21) Anmeldenummer : **84100346.0**

(22) Anmeldetag : 14.01.84

(54) **Probeentnahmegerät für ein Milchmengenmessgerät für Melkanlagen.**

(30) Priorität : 04.03.83 DE 3307665

(43) Veröffentlichungstag der Anmeldung :
12.09.84 Patentblatt 84/37

(45) Bekanntmachung des Hinweises auf die Patenterteilung : 12.11.86 Patentblatt 86/46

(84) Benannte Vertragsstaaten :
FR GB IT NL SE

(56) Entgegenhaltungen :
EP-A- 0 023 449
DE-A- 3 103 669
NL-A- 7 901 804

(73) Patentinhaber : **Westfalia Separator AG**
**Werner-Habig-Strasse 1 Postfach 3720**
**D-4740 Oelde 1 (DE)**

(72) Erfinder : **Die Erfinder haben auf Ihre Nennung**
**verzichtet**

(74) Vertreter : **Stracke, Alexander, Dipl.-Ing. et al**
**Patentanwälte Dipl.-Ing. Loesenbeck Dipl.-Ing.**
**Stracke Jöllenbecker Strasse 164 Postfach 5605**
**D-4800 Bielefeld 1 (DE)**

## Beschreibung

Die Erfindung bezieht sich auf ein Probeentnahmegerät für ein Milchmengenmeßgerät für Melkanlagen, mit dem Teilmengenmessungen durchführbar sind und das eine unterdruckbeaufschlagte Trennkammer sowie eine Meßkammer aufweist und bei dem die Trennkammer mit einer durch einen Ventilkolben verschließbaren Auslaßöffnung versehen ist.

Es ist ein Milchmengenmeßgerät der genannten Art bekannt (DE-OS 31 03 669), bei dem bei geöffneter Auslaßöffnung die Milch in eine unterdruckbeaufschlagte Sammelleitung fließt, ohne daß eine Probenahme durchgeführt wird.

Ferner ist ein Probeentnahmegerät für Melkanlagen aus der EP-A-23 449 bekannt, dessen Probegefäß an die Unterseite einer Trennkammer angeschloßen ist. Durch diesen Anschluß wird Milch nur vom unteren Teil der Trennkammer für Proben entnommen. Da die Milch im unteren Teil der Trennkammer weniger Fett als die Milch im oberen Teil der Trennkammer enthält, ist eine genaue Probenahme nicht gewährleistet.

Der Erfindung liegt die Aufgabe zugrunde, ein Probeentnahmegerät für ein Milchmengenmeßgerät der genannten Art so zu gestalten, daß das Probeentnahmegerät keine Vakuumschwankungen verursacht, die Teilmengenmessungen im Milchmengenmeßgerät nicht beeinflußt werden und die im Sammelgefäß gewonnene Probe sich aus Teilmilchmengen zusammensetzt, deren Anzahl der im Milchmengenmeßgerät durchgeführten Teilmengenmessungen entspricht.

Diese Aufgabe wird nach der Erfindung dadurch gelöst, daß der der Auslaßöffnung zugeordnete Ventilkolben eine zur Trennkammer geöffnete Düsenbohrung und einen sich anschließenden Düsenkanal aufweist, der im Bereich des der Düsenbohrung abgewandten Endes mit einer Ablauföffnung ausgerüstet ist, die in der Verschlußstellung des Ventilkolbens geschlossen ist und in der Öffnungsstellung des Ventilkolbens in eine zum Milchsammelraum des Probeentnahmegerätes führende Leitung einmündet.

Mit dem erfindungsgemäßen Probeentnahmegerät wird jeweils bei geöffneter Auslaßöffnung des Milchmengenmeßgerätes aus der zur Sammelleitung fließenden Milchmenge eine Teilmenge entnommen, so daß die in den Milchsammelraum des Probeentnahmegerätes gelangende Milch sich aus verteilt über die Melkzeit entnommenen Teilmengen zusammensetzt.

Die beim Melkvorgang im ersten Zeitraum ermolkene Milch enthält weit weniger Fett als die im letzten Zeitraum gewonnene Milch.

Durch das erfindungsgemäße Probeentnahmegerät wird gewährleistet, daß bei einer portionsweise gemessenen Milchmenge aus jeder Portion eine der Größe der Portion entsprechende Teilmenge entnommen wird. Da die Portionsgröße von der Öffnungszeit eines Entleerungsventils bestimmt wird, wird während der Entleerungszeit jeweils ein proportionaler Anteil der entleerten Milchmenge als Sammelprobe genommen.

Die im Milchsammelraum des Probeentnahmegerätes befindliche Milch muß vor der Abfüllung in die Probeflaschen gemischt werden, damit die Inhaltsstoffe in der Milch gleichmäßig verteilt sind.

Da für die eigentliche Untersuchung nur eine geringe Milchmenge, z. B. 20 cm³, benötigt wird, kann die darüber hinaus im Milchsammelraum des Probeentnahmegerätes befindliche Milchmenge auf einfache Weise zu der bereits im Kühltank befindlichen Milch gefördert werden.

Bei einer vorteilhaften Ausführungsform des erfindungsgemäßen Probeentnahmegerätes ist das den Milchsammelraum aufweisende Gefäß mit einem ortsfesten Oberteil versehen, an dem ein den Deckel des Gefäßes bildendes Unterteil drehbar gelagert ist. Das Oberteil ist mit zwei Anschlußstutzen sowie mit mindestens einer Steuerbohrung und das Unterteil mit mindestens zwei Kanälen ausgerüstet, die mit den Anschlußstutzen in eine fluchtende Stellung (Melkstellung) gebracht werden können. Durch Verdrehen des Unterteils gegenüber dem Oberteil sind verschiedene Betriebsvorgänge, nämlich die Probenahme, das Mischen der im Sammelraum des Probeentnahmegerätes vorhandenen Milch, das Absaugen der nicht benötigten Milchmenge aus dem Sammelraum sowie das Entleeren des Sammelraumes erziel-bar.

Ein Ausführungsbeispiel des erfindungsgemäßen Probeentnahmegerätes ist in den Zeichnungen dargestellt und wird im folgenden beschrieben. Es zeigen :

Figur 1 ein Milchmengenmeßgerät mit angehängtem Probeentnahmegerät, teilweise im Vertikalschnitt, wobei die Auslaßöffnung des Milchmengenmeßgerätes geschlossen ist,

Figur 2 das Milchmengenmeßgerät mit angehängtem Probeentnahmegerät nach der Fig. 1 mit geöffneter Auslaßöffnung,

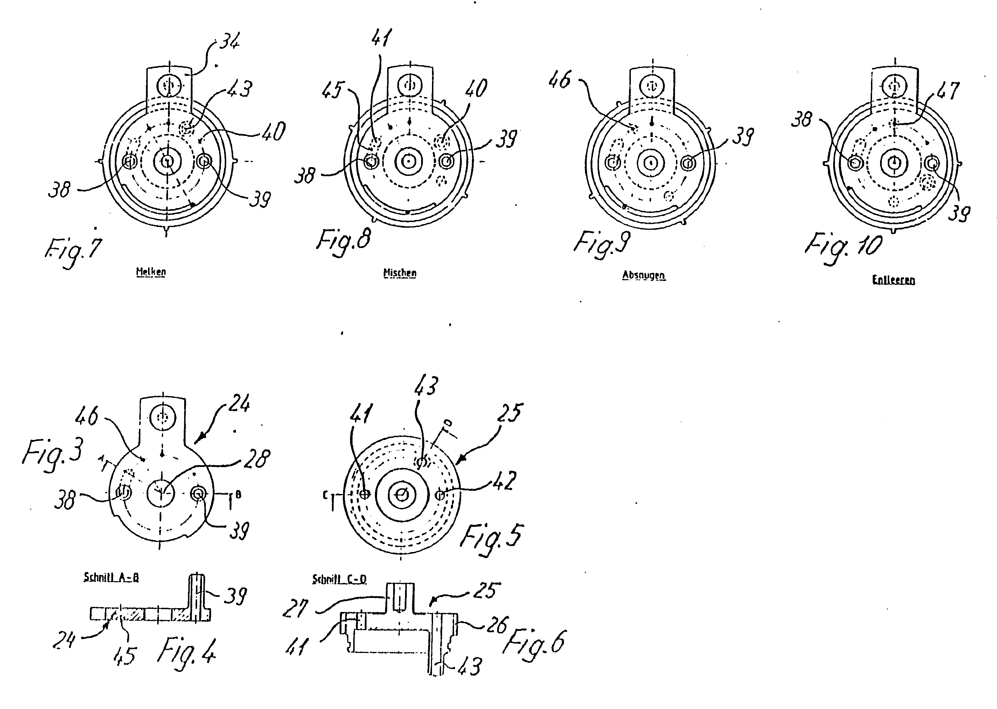

Figur 3 das Oberteil des den Milchsammelraum bildenden Gefäßes im Grundriß,

Figur 4 einen Schnitt nach der Linie A-B in Fig. 3,

Figur 5 das den Deckel des Gefäßes bildende Unterteil im Grundriß,

Figur 6 einen Schnitt nach der Linie C-D in Fig. 5,

Figur 7 bis 10 das Oberteil und das drehbar am Oberteil gelagerte Unterteil im Grundriß in verschiedenen Betriebsstellungen.

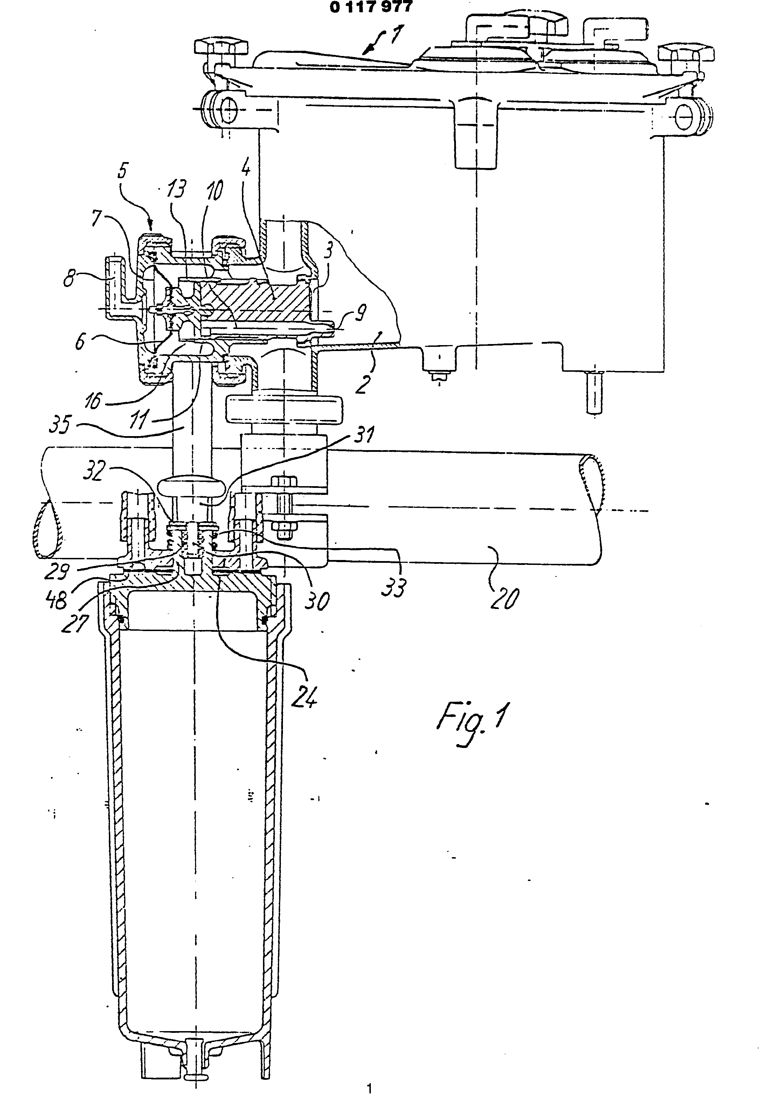

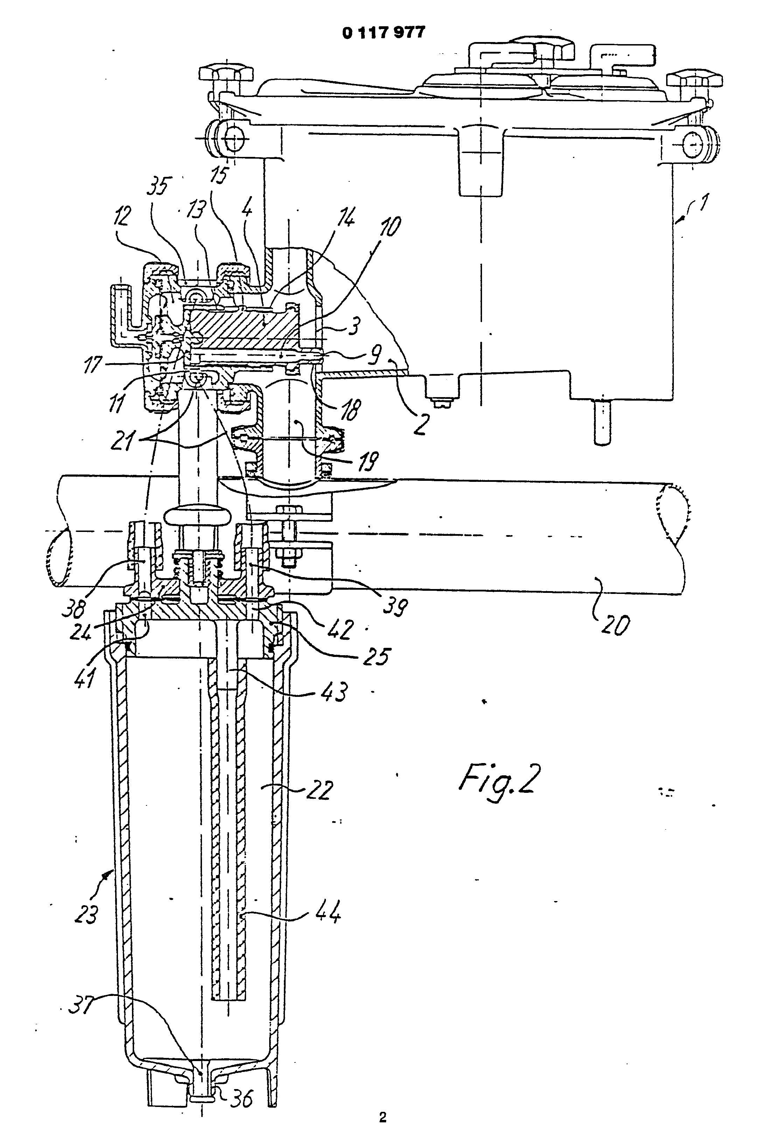

Das in den Fig. 1 und 2 dargestellte Milchmengenmeßgerät 1 ist mit einer unterdruckbeaufschlagten Trennkammer 2 ausgerüstet, der das abgemolkene Milch-Luft-Gemisch zugeführt wird. Von der Trennkammer gelangt die Milch in eine Meßkammer, in der Teilmengenmessungen durchgeführt werden. Das Milchmengenmeßgerät ist mit einer Auslaßöffnung 3 versehen, die durch einen Ventilkolben 4 verschlossen werden kann. In dem dargestellten Ausführungsbeispiel ist der Ventilkolben an einer im Ventilgehäuse 5 eingespannten Membran 6 befestigt, die eine Steuerkammer 7 an der Innenseite begrenzt, die über einen Stutzen 8 mit Luft atmosphärischen Druckes oder mit Unterdruck beaufschlagt werden kann. Das Öffnen und Schließen der Auslaßöffnung 3 kann mittels eines elektronischen Steuergerätes vorgenommen werden, das mit Sensoren verbunden ist, die in Abstand voneinander in der Meßkammer angeordnet sind.

Der Ventilkolben 4 weist eine zur Trennkammer 2 geöffnete Düsenbührung 9 auf, an der sich ein Düsenkanal 10 anschließt. Im Bereich des der Düsenbohrung 9 abgewandten Endes ist der Düsenkanal mit einer Ablauföffnung 11 versehen, die in der Verschlußstellung des Ventilkolbens 4 abgesperrt ist und in der in der Fig. 2 aufgezeigten Öffnungsstellung des Ventilkolbens 4 in eine unterdruckbeaufschlagte Ventilkammer 12 einmündet. Der Ventilkolben 4 ist in dem dargestellten Ausführungsbeispiel in einer Hülse 13 gleitbar gelagert, die eine schlitzförmige Führungsbahn 14 aufweist, in die ein Nocken 15 des Ventilkolbens 4 eingreift. Hierdurch wird der Ventilkolben gegen eine Verdrehung gegenüber der Hülse 13 gesichert. An dem der Trennkammer 2 abgewandten Ende ist die Hülse mit einem Schlitz 16 versehen, in dessen Bereich die Ablauföffnung 11 des Düsenkanals 10 in der Öffnungsstellung des Ventilkolbens 4 liegt.

Der Ventilkolben 4 ist mit einer Endkappe 17 ausgerüstet, durch die die hintere stirnseitige Öffnung des Düsenkanals 10 verschlossen wird. Die Endkappe 17 ist an der Membran 6 befestigt.

Die Düsenbohrung 9 ist in dem dargestellten Ausführungsbeispiel in einem von der der Trennkammer 2 zugewandten Stirnseite des Ventilkolbens 4 vorspringenden Zapfen 18 angeordnet.

Bei geöffneter Auslaßöffnung 3 fließt aus dem Milchmengenmeßgerät 1 eine Milchmenge durch die Leitung 19 in die unterdruckbeaufschlagte Sammelleitung 20. Eine Teilmenge strömt durch die Düsenbohrung 9 in den Düsenkanal 10 und gelangt über die Ablauföffnung 11 in eine Leitung 21 und wird durch diese Leitung in den Milchsammelraum 22 des Gefäßes 23 geführt.

Nachdem der Ventilkolben 4 in die Verschlußstellung gebracht worden ist, die in der Fig. 1 aufgezeigt ist, wird durch die Hülse 13 die Ablauföffnung 11 abgesperrt. Es beginnt eine neue Teilmengenmessung im Milchmengenmeßgerät.

Das Gefäß 23 ist mit einem ortsfesten Oberteil 24 versehen, an dem ein Unterteil 25 drehbar gelagert ist, daß den Deckel des Gefäßes 23 bildet und den Milchsammelraum 22 nach oben begrenzt. Das Unterteil 25 ist mit einem Außengewinde 26 in ein Innengewinde des Gefäßes 23 geschraubt. Zur drehbaren Lagerung des Unterteils 25 ist dieses mit einem mittigen Zapfen 27 versehen, der sich durch eine mittige Bohrung 28 des Oberteils erstreckt. Der Zapfen 27 ist als Hohlzapfen ausgebildet und weist eine mit einem Innengewinde ausgerüstete Buchse 29 auf, in die der Gewindeschaft 30 einer Schraube 31 geschraubt wird. Zwischen einer Unterlegscheibe 32 und dem Oberteil 24 ist eine zylindrische Schraubenfeder 33 angeordnet. Hierdurch wird eine kraftschlüssige Verbindung zwischen dem Oberteil 24 und dem Unterteil 25 geschaffen, wobei zwischen den zusammenwirkenden Flächen des Oberteils und des Unterteils eine Gleitdichtung vorgesehen ist, die als Scheibe 48 ausgebildet sein kann.

Das Oberteil 24 ist mit einem seitlichen Ausleger 34 ausgerüstet, an dem ein Aufhängebügel 35 befestigt ist, der in eine Ringnut des Ventilgehäuses 5 eingehängt ist.

Im Boden des Gefäßes 23 ist ein Entleerungskanal 36 vorgesehen, in dem ein unter Vakuumeinfluß in die Verschlußstellung bewegbarer Ventilkörper 37 gleitbar gelagert ist.

Das Oberteil 24 ist mit zwei Anschlußstutzen 38, 39 sowie mit mindestens einer Steuerbohrung 40 ausgerüstet. Das Unterteil 25 ist mit mindestens zwei Kanälen 41, 42 versehen, die mit den Anschlußstutzen 38, 39 in eine fluchtende Stellung gebracht werden können, die in der Fig. 2 aufgezeigt ist und in der eine Probenahme stattfindet.

Durch Verdrehen des Unterteils gegenüber dem Oberteil können verschiedene Betriebsvorgänge erzielt werden, die im folgenden unter Bezugnahme auf die Fig. 7 bis 10 erläutert werden.

Bei dem dargestellten Ausführungsbeispiel weist das Unterteil 25 zusätzlich zu den Kanälen 41 und 42 einen weiteren Kanal auf, der als in den Sammelraum 22 ragender Stutzen 43 ausgebildet ist und an dem ein bis in den unteren Bereich des Sammelraumes 22 ragender Schlauch 44 befestigt ist.

Nach der Beendigung des Melkens und somit der Probenahme muß die im Sammelraum 22 des Gefäßes befindliche Milch gemischt werden. Dies wird durch Einspeisung von Luft unter atmosphärischem Druck vorgenommen. Das Unterteil 25 wird durch Verdrehen gegenüber dem Oberteil 24 in die in der Fig. 8 aufgezeigte Lage gebracht. In dieser Lage ist der Kanal 41 des Unterteils über ein Kanalsegment 45 im Oberteil mit dem Anschlußstutzen 38 des Oberteils verbunden, der unterdruckbeaufschlagt ist. Die Milchzufuhr durch den Anschlußstutzen 39 ist abgesperrt. Die Steuerbohrung 40 des Oberteils liegt im Bereich des in den Sammelraum ragenden Stutzens 43 des Unterteils. Durch die Steuerbohrung 40 strömt Luft in den Sammelraum 22, durch die eine intensive Durchmischung der Milch vorgenommen wird.

Um überschüssige Milch aus dem Sammelraum 22 abzusaugen, wird das Unterteil durch eine weitere Verdrehung im Uhrzeigersinn in die Betriebslage gebracht, die in der Fig. 9 aufgezeigt ist. Eine Steuerbohrung 46 des Oberteils liegt im Bereich des Kanals 41 des Unterteils. Durch diese Steuerbohrung strömt Luft in den Sammelraum 22.

Der mit dem Schlauch 44 versehene Stutzen 43 des Unterteils fluchtet mit dem Anschlußstutzen 39 des Oberteils, der unterdruckbeaufschlagt ist, und zwar über die zur Ventilkammer 12 führende Leitung 21. Über diese Leitung und über die genannte Ventilkammer gelangt die abgesaugte Milch in die Sammelleitung 20.

In der Fig. 10 ist die Betriebsstellung des Unterteils zum Oberteil dargestellt, in der das Gefäß 23 entleert wird.

Die Anschlußstutzen 38 und 39 des Oberteils sind durch das Unterteil 25 abgesperrt. Durch eine Steuerbohrung 47 des Oberteils, die im Bereich des Kanals 41 des Unterteils 25 liegt, strömt in den Sammelraum 22 Luft ein, so daß der Ventilkörper 37 sich in die Öffnungsstellung bewegt und die im Sammelraum 22 sich befindende Milch ablaufen kann. Bei dem erfindungsgemäßen Probeentnahmegerät können somit die verschiedenen Betriebsvorgänge mit einfachen konstruktiven Maßnahmen erreicht werden.

**Patentansprüche**

1. Probeentnahmegerät für ein Milchmengenmeßgerät für Melkanlagen, mit dem Teilmengenmessungen durchführbar sind und das eine unterdruckbeaufschlagte Trennkammer sowie eine Meßkammer aufweist und mit einer durch einen Ventilkolben verschließbaren Auslaßöffnung versehen ist, dadurch gekennzeichnet, daß der Ventilkolben (4) eine zur Trennkammer (2) geöffnete Düsenbohrung (9) und einen sich anschließenden Düsenkanal (10) aufweist, der im Bereich des der Düsenbohrung abgewandten Endes mit einer Ablauföffnung (11) ausgerüstet ist, die in der Verschlußstellung des Ventilkolbens geschlossen ist und in der Öffnungsstellung des Ventilkolbens in eine zum Milchsammelraum 22 des Probeentnahmegerätes führende Leitung (21) einmündet.

2. Probeentnahmegerät nach Anspruch 1, dadurch gekennzeichnet, daß der Ventilkolben (4) in einer Hülse (13) gleitbar gelagert ist, die an dem der Trennkammer (2) abgewandten Ende mit einem Schlitz (16) versehen ist, in dessen Bereich die Ablauföffnung (11) des Düsenkanals (10) in der Öffnungsstellung des Ventilkolbens (4) liegt.

3. Probeentnahmegerät nach Anspruch 2, dadurch gekennzeichnet, daß der Schlitz (16) der Hülse (13) in eine unterdruckbeaufschlagte Ventilkammer (12) einmündet, von der eine milchführende Leitung (21) und eine Vakuumleitung sich zum Sammelraum (22) erstrecken.

4. Probeentnahmegerät nach einem der Ansprüche 1 bis 3, dadurch gekennzeichnet, daß an dem Ventilkolben (4) an der der Düsenbohrung (9) abgewandten Seite eine eine stirnseitige Öffnung des Düsenkanals (10) verschließende Endkappe (16) befestigt ist, die an einer im Ventilgehäuse (5) eingespannten Membran (6) festgelegt ist.

5. Probeentnahmegerät nach einem der Ansprüche 1 bis 4, dadurch gekennzeichnet, daß die Düsenbohrung (9) in einem von der der Trennkammer (2) zugewandten Stirnseite des Ventilkolbens (4) vorspringenden Zapfen (18) angeordnet ist.

6. Probeentnahmegerät nach einem der Ansprüche 1 bis 5, dadurch gekennzeichnet, daß das den Milchsammelraum (22) aufweisende Gefäß (23) mit einem ortsfesten Oberteil (24) versehen ist, an dem ein den Deckel des Gefäßes bildendes Unterteil (25) drehbar gelagert ist, das Oberteil (24) mit zwei Anschlußstutzen (39, 38) sowie mindestens einer Steuerbohrung und das Unterteil (25) mit mindestens zwei Kanälen (41, 42) ausgerüstet ist, die mit den Anschlußstutzen in eine fluchtende Stellung bringbar sind und daß durch Verdrehen des Unterteils gegenüber dem Oberteil verschiedene Betriebsvorgänge erzielbar sind.

7. Probeentnahmegerät nach Anspruch 6, dadurch gekennzeichnet, daß ein Kanal des Unterteils (25) als in den Sammelraum (22) ragender Stutzen (43) ausgebildet ist, an dem eine bis in den unteren Bereich des Sammelraumes sich erstreckende Leitung, vorzugsweise ein Schlauch, befestigt ist.

8. Probeentnahmegerät nach Anspruch 6 oder 7, dadurch gekennzeichnet, daß zum Mischen der im Sammelraum (22) befindlichen Milch durch Verdrehen des Unterteils (25) die Milchzufuhr zum Sammelraum abgesperrt und ein Kanal des Unterteils in eine fluchtende Lage mit der Steuerbohrung des Oberteils gebracht wird, durch die atmosphärische Luft in den Sammelraum einströmt, wobei der zweite Kanal (41) des Unterteils (25) über ein Kanalsegment (45) des Oberteils mit der zum Oberteil führenden Vakuumleitung verbunden bleibt.

9. Probeentnahmegerät nach einem der vorhergehenden Ansprüche, dadurch gekennzeichnet, daß im Boden des Sammelgefäßes (23) ein Entleerungskanal (36) vorgesehen ist, in dem ein unter Vakuumeinfluß in die Verschlußstellung bewegbarer Ventilkörper (37) gleitbar gelagert ist.

10. Probeentnahmegerät nach Anspruch 9, dadurch gekennzeichnet, daß zum Entleeren des Sammelgefäßes (22) das Unterteil (25) zum Oberteil (24) in einer Lage arretiert ist, in der die Anschlußstutzen (38, 39) des Oberteils abgesperrt sind und ein Kanal des Unterteils mit einer Steuerbohrung des Oberteils fluchtet, durch die atmosphärische Luft in den Sammelraum (22) einströmt.

11. Probeentnahmegerät nach einem der vorhergehenden Ansprüche, dadurch gekennzeichnet, daß zum Absaugen der für die Probenahme überschüssigen Milch aus dem Sammelraum (22) das Unterteil (25)l zum Oberteil (24) in einer Lage arretiert ist, in der die vom Unterteil in den Sammelraum sich erstreckende Leitung mit einem Anschlußstutzen des Oberteils fluchtet und eine Steuerbohrung des Oberteils im Bereich eines Kanals des Unterteils liegt.

12. Probeentnahmegerät nach einem der Ansprüche 1 bis 11, dadurch gekennzeichnet, daß am Oberteil (24) ein Aufhängebügel (35) befestigt ist, der in eine Ringnut des Ventilgehäuses (5) einhängbar ist.

13. Probeentnahmegerät nach Anspruch 6, dadurch gekennzeichnet, daß zwischen dem Oberteil (25) und dem Unterteil (24) eine eine Gleitdichtung bildende Scheibe (48) angeordnet ist.

**Claims**

1. A sampling device for a milk flow measuring apparatus for milking installations, with which part-quantity measuring operations can be carried out and which has a separating chamber subjected to a reduced pressure, and a measuring chamber, and which is provided with an outlet opening which can be closed by a valve piston, characterised in that the valve piston (4) has a nozzle bore (9) which is opened relative to the separating chamber (2), and has an adjoining nozzle passage (10) which is provided in the region of the end remote from the nozzle bore with a discharge opening (11) which is closed in the closure position of the valve piston and which iń the open position of the valve piston opens into a conduit (21) leading to the milk collecting chamber (22) of the sampling device.

2. A sampling device according to claim 1 characterised in that the valve piston (4) is mounted slidably in a sleeve (13) which is provided with a slot (16) at the end remote from the separating chamber (2), the discharge opening (11) of the nozzle passage (10) being disposed in the region of said slot (16), in the open position of the valve piston (4).

3. A sampling device according to claim 2 characterised in that the slot (16) in the sleeve (13) opens into a valve chamber (12) which is subjected to a reduced pressure and from which a milk-carrying conduit (21) and a vacuum conduit extend to the collecting chamber (22).

4. A sampling device according to one of claims 1 to 3 characterised in that secured to the valve piston (4) at the side remote from the nozzle bore (9) is an end cap (17) which closes an end opening of the nozzle passage (10) and which is fixed to a diaphragm (6) which is clamped in the valve housing (5).

5. A sampling device according to one of claims 1 to 4 characterised in that the nozzle bore (9) is disposed in a projection portion (18) projecting from the face of the valve piston (4), which is towards the separating chamber (2).

6. A sampling device according to one of claims 1 to 5 characterised in that the vessel (23) having the milk collecting chamber (22) is provided with a stationary upper portion (24) on which a lower portion (25) forming the lid of the vessel is rotatably mounted, the upper portion (24) is provided with two connecting portions (39, 38) and at least one control bore and the lower portion (25) is provided with at least two passages (41, 42) which can be brought into a position of alignment with the connecting portions, and that various operating procedures can be achieved by rotation of the lower portion relative to the upper portion.

7. A sampling device according to claim 6 characterised in that a passage in the lower portion (25) is in the form of a connection (43) which projects into the collecting chamber (22), a conduit, preferably a hose, which extends into the lower region of the collecting chamber being secured to said connection (43).

8. A sampling device according to claim 6 or claim 7 characterised in that, for the purposes of mixing of the milk in the collecting chamber (22), the feed of milk to the collecting chamber is shut off by rotation of the lower portion (25) and a passage in the lower portion is brought into a position of alignment with the control bore in the upper portion, through which atmospheric air flows into the collecting chamber, wherein the second passage (41) in the lower portion (25) remains in communication with the vacuum conduit leading to the upper portion, by way of a passage segment (45) in the upper portion.

9. A sampling device according to one of the preceding claims characterised in that provided in the bottom of the collecting vessel (23) is an emptying passage (36) in which there is slidably mounted a valve member (37) which can be moved into the closure position under the effect of vacuum.

10. A sampling device according to claim 9 characterised in that, for the purposes of emptying of the collecting vessel (22), the lower portion (25) is arrested relative to the upper portion (24) in a position in which the connecting portions (38, 39) of the upper portion are shut off and a passage in the lower portion is aligned with a control bore in the upper portion, through which atmospheric air flows into the collecting chamber (22).

11. A sampling device according to one of the preceding claims characterised in that, for the purposes of sucking the milk which is in excess for the sampling operation, out of the collecting chamber (22), the lower portion (23) is arrested relative to the upper portion (24) in a position in which the conduit which extends from the lower portion into the collecting chamber is aligned with a connecting portion of the upper portion and a control bore of the upper portion lies in the region of a passage in the lower portion.

12. A sampling device according to one of claims 1 to 11 characterised in that a suspension loop (35) is secured to the upper portion (24), and can be engaged into an annular groove in the valve housing (5).

13. A sampling device according to claim 6 characterised in that a disc (48) forming a sliding sealing means is arranged between the upper portion (25) and the lower portion (24).

**Revendications**

1. Dispositif de prélèvement d'échantillon dans un appareil de mesure de quantité de lait pour les installations de traite avec lequel on réalise des mesures de quantités partielles et qui comporte une chambre de séparation dans laquelle règne une dépression, ainsi qu'une chambre de mesure et qui est équipé d'une ouverture d'évacuation obturable par un piston à soupape, caractérisé en ce que le piston à soupape (4) présente un trou de busette (9), ouvert dans la chambre de séparation (2), auquel est raccordé un canal d'écoulement (10) qui, dans la région de l'extrémité opposée au trou de busette, est équipé d'une ouverture d'écoulement (11) qui est fermée en position d'obturation du piston à soupape et débouche, en position d'ouverture du piston à soupape, dans une conduite (21) conduisant à la chambre collectrice de lait (22) du dispositif de prélèvement d'échantillon.

2. Dispositif de prélèvement d'échantillon selon la revendication 1, caractérisé en ce que le piston à soupape (4) est logé coulissant dans un manchon (13) qui, sur l'extrémité opposée à la chambre de séparation (2) est muni d'une fente (16) dans la zone de laquelle est placée l'ouverture d'écoulement (11) du canal (10) en position d'ouverture du piston à soupape (4).

3. Dispositif de prélèvement d'échantillon selon la revendication 2, caractérisé en ce que la fente (16) du manchon (13) débouche dans une boîte de distribution (12) dans laquelle règne une dépression d'où partent une conduite (21) amenant le lait et une canalisation de vide jusqu'à la chambre collectrice (22).

4. Dispositif de prélèvement d'échantillon selon l'une des revendications 1 à 3, caractérisé en ce qu'une calotte terminale (16) fermant une ouverture de bout du canal (10) est fixée sur le piston à soupape (4) sur la paroi opposée au trou de busette (9), calotte qui est fixée sur une membrane (6) tendue dans la cage de soupape (5).

5. Dispositif de prélèvement d'échantillon selon l'une des revendications 1 à 4, caractérisé en ce que le trou de busette (9) est placé dans un tourillon (18) en saillie depuis la face avant du piston à soupape (4) opposée à la chambre de séparation (2).

6. Dispositif de prélèvement d'échantillon selon l'une des revendications 1 à 5, caractérisé en ce que le récipient constituant la chambre collectrice de lait (22) est équipé d'une partie supérieure fixe (24) sur laquelle une partie inférieure formant le couvercle du récipient est montée pivotante, la partie supérieure (24) est équipée de deux ajutages (39, 38) ainsi qu'au moins un orifice de distribution, et la partie inférieure (25) est équipée d'au moins deux canaux (41, 42) qui sont susceptibles d'être placés en position alignée avec les ajutages, et en ce que, en faisant tourner la partie inférieure par rapport à la partie supérieure, on peut obtenir différentes allures de marche.

7. Dispositif de prélèvement d'échantillon selon la revendication 6, caractérisé en ce qu'un canal de la partie inférieure (25) forme un tuyau (43) sortant dans la chambre collectrice (22), tuyau sur lequel est fixée une canalisation, de préférence un tuyau flexible s'avançant dans la partie inférieure de la chambre collectrice.

8. Dispositif de prélèvement d'échantillon selon la revendication 6 ou la revendication 7, caractérisé en ce que l'amenée du lait à la chambre collectrice est obturée lors du mélange du lait se trouvant dans la chambre collectrice (22) par rotation de la partie inférieure (25) et un canal de la partie inférieure est placé en alignement avec l'orifice de distribution de la partie supérieure, l'écoulement dans la chambre collectrice se fait par la pression atmosphérique, le deuxième canal (41) de la partie inférieure (25) restant relié par une section de canal (45) de la partie supérieure à la canalisation de vide conduisant à cette partie supérieure.

9. Dispositif de prélèvement d'échantillon selon l'une des revendications précédentes, caractérisé en ce qu'il est prévu, dans le corps du récipient collecteur (23), un canal de purge (36) dans lequel est logé un corps de soupape (37), mobile, susceptible de se déplacer sous l'influence du vide dans la position de fermeture.

10. Dispositif de prélèvement d'échantillon selon la revendication 9, caractérisé en ce que, pour la purge du récipient collecteur (22), la partie inférieure (25) est bloquée par rapport à la partie supérieure (24) dans une position où les ajutages (38, 39) de la partie supérieure sont bloqués et où un canal de la partie inférieure est aligné avec un orifice de distribution de la partie supérieure, par lequel l'air atmosphérique est admis dans la chambre collectrice (22).

11. Dispositif de prélèvement d'échantillon selon l'une des revendications précédentes, caractérisé en ce que, pour l'aspiration à partir de la chambre collectrice (22) du lait en surplus lors de la prise d'échantillon, la partie inférieure (25) est bloquée, par rapport à la partie supérieure, dans une position selon laquelle la canalisation qui sort depuis la partie inférieure dans la chambre collectrice est alignée avec un ajutage de la partie supérieure et un orifice de distribution de la partie supérieure se trouve dans la zone d'un canal de la partie inférieure.

12. Dispositif de prélèvement d'échantillon selon l'une des revendications 1 à 11, caractérisé en ce que, à la partie supérieure (24), est fixée une barette de suspension (35), qui peut être accrochée dans une rainure de la cage de soupape (5).

13. Dispositif de prélèvement d'échantillon selon la revendication 6, caractérisé en ce qu'un disque (48) formant une étanchéité par glissement est disposé entre la partie supérieure (25) et la partie inférieure (24).

Fig.1

0 1 1 7 9 7 7

Fig.2

2

Fig.7 — Melken

Fig.8 — Mischen

Fig.9 — Absaugen

Fig.10 — Entleeren

Fig.3

Fig.4 — Schnitt A–B

Fig.5

Fig.6 — Schnitt C–D

0 117 977